# EUROPEAN PATENT APPLICATION

(11) **EP 2 508 175 A1**
(43) Date of publication of application: **10.10.2012**
(21) Application number: 11161641.3
(22) Date of filing: 08.04.2011
(51) Int. Cl.: A61K 9/20, A61K 9/28, A61K 31/222

(54) **Pharmaceutical composition comprising fesoterodine or a salt or a solvate thereof**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to a new pharmaceutical composition of fesoterodine or a salt or a solvate thereof, in particular to a dry formulation. The present invention relates to a new process for the preparation of dry formulation of fesoterodine or a salt or a solvate thereof. The pharmaceutical composition is particularly useful as a medicament, especially for the treatment of urinary incontinence.

## Description

### Field of the Invention

The present invention relates to a pharmaceutical composition of fesoterodine or a salt or a solvate thereof, in particular to a dry formulation. The present invention relates to a process for the preparation of dry formulation of fesoterodine or a salt or a solvate thereof. The pharmaceutical composition is particularly useful as a medicament, especially for the treatment of urinary incontinence.

### Description of Background Art

Fesoterodine ([2-[(1R)-3-(Di(propan-2-yl)amino)-1-phenylpropyl]-4-(hydroxymethyl)phenyl] 2-methylpropanoate) is a muscarinic receptor antagonist used for the treatment of overactive bladder including urinary incontinence. Fesoterodine substance was disclosed in WO 98/43942 and WO 99/58478, while salts of fesoterodine were described in EP 1230209.

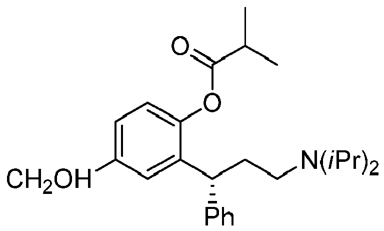

Fesoterodine is a tolterodine (3-(2-hydroxy-5-methylphenyl)-N,N-diisopropyl-3-phenylpropylamine) prodrug that is converted to an active molecule hydroxy metabolite 2-(3-(diisopropylamino)-1-phenylpropyl)-4-(hydroxymethyl)phenol (hydroxytolterodine) in the body. However, fesoterodine substance is prone to conversion to 5-hydroxymethyltolterodine under humid environment and at increased temperature. Said degradation of fesoterodine to 5-hydroxymethyltolterodine in the pharmaceutical formulation is undesirable. Therefore, there is a need to provide a pharmaceutical composition comprising fesoterodine that is stable against fesoterodine degradation over an extended period of time.

So far, two solutions regarding fesoterodine stability in the pharmaceutical composition have been presented. In WO 2007/141298 pharmaceutical excipients such as xylitol, sorbitol, polydextrose, isomalt and combinations thereof were found to be able to significantly slow down the degradation of fesoterodine under stress conditions. WO 2010/043408 discloses a microencapsuled fesoterodine composition which, distinct from a homogenous mixture of fesoterodine-particle with a matrix, is composed of a particle containing fesoterodine and a shell surrounding the fesoterodine-containing particle. However, the prior art proposed compositions require a certain amount of stabilizers for preventing degradation (cf. WO 2007/141298), or a complex-structured pharmaceutical composition (cf. WO 2010/043408).

Therefore, there is an unmet need for new pharmaceutical composition comprising fesoterodine that are stable against fesoterodine degradation over an extended period of time.

### Summary of the invention

The aspects, advantageous features and preferred embodiments of the present invention summarized in the following items, respectively alone or in combination, further contribute to solving the object of the invention:
1. A dry formulation of fesoterodine or a salt or a solvate thereof, comprising said fesoterodine or a salt or a solvate thereof homogeneously admixed with at least one excipient in said dry formulation, and wherein said dry formulation is free of a stabilizer selected from xylitol, sorbitol, polydextrose, isomalt and dextrose.
2. The dry formulation according to item 1, wherein an optional liquid, when used, was limited during the dry formulation process of preparation at a maximum of 10% liquid relative to the composition subjected to formulation preferably the dry formulation is obtainable by a process carried out substantially without the presence of water.
3. The dry formulation according to any one of the preceding items, wherein a residual amount of water or moisture in the dry formulation is 4.0 wt.-% or below, preferably is 2.0 wt.-% or below, measured by loss on drying.
4. The dry formulation according to any one of the preceding items, wherein none of excipients or additives added to the formulation are combined with agglomeration liquid, and all the excipients or additives are added in the form of dry material or powders.
5. The dry formulation according to anyone of the preceding items, wherein the content of inactive excipients in the homogeneous admixture with of fesoterodine or a salt or a solvate thereof constitutes a majority, preferably at least 75%, more preferably at least 90% of the total dry formulation.
6. The dry formulation according to anyone of the preceding items, further comprising pharmaceutically acceptable excipients selected from fillers, glidants and lubricants present in said dry formulation.
7. The dry formulation according to any of preceding items, comprising relative to the total amount of the dry formulation:
   (i) 0.5-15% fesoterodine or a salt or a solvate thereof,
   (ii) 5-99% filler selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof,
   (iii) 0.5-5 % glidant,
   (iv) 0.5-5% lubricant.
8. The dry formulation according to any of preceding items comprising
   (i) 2-5% fesoterodine or a salt or a solvate thereof,
   (ii) 20-95% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof,
   (iii) 2-5% glidants and
   (iv) 2-5% lubricants.
9. The dry formulation according to any of preceding items comprising
   (i) 2-3% fesoterodine or a salt or a solvate thereof,
   (ii) 40-95% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof,
   (iii) 2-3% glidants and
   (iv) 3-4% lubricants.
10. The dry formulation according to any one of the preceding items, wherein an excipient, homogeneously admixed with the fesoterodine or a salt or a solvate thereof in said dry formulation, is a polymer, preferably a controlled release polymer.
11. The dry formulation according to item 10, wherein the controlled release polymer is selected from the group consisting of hydroxypropyl methylcellulose, methylcellulose hydroxyethylcellulose, hydroxypropylcellulose, carboxymethylcellulose, ethylcellulose, polyethyleneoxide, carrageenan, agar, alginic acid, pectin and a mixtures thereof, preferably hydroxypropyl methylcellulose.
12. The dry formulation according to item 11, wherein the controlled release polymer is hydroxypropyl methylcellulose or a combination of at least two different viscosity grades of hydroxypropyl methylcellulose.
13. The dry formulation according to anyone of items 10 to 12, which comprises 15-65 % of said polymer, preferably the controlled release polymer, relative to the total amount of the dry formulation.
14. The dry formulation according to item 13, comprising about 40-50% of the polymer.
15. The dry formulation according to any of items 10 to 14, comprising relative to the total amount of the dry formulation:
   (i) 0.5-10% fesoterodine fumarate,
   (ii) 5-85% filler selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof"
   (iii) 15-65% controlled release polymers, selected from a group consisting of hydroxypropyl methylcellulose, methylcellulose and mixture thereof,
   (iv) 0.5-5% % glidant, preferably talc,
   (v) 0.5-5% % lubricant, preferably glyceryl behenate.
16. The dry formulation according to any of preceding items further comprising
   (i) 2-5% fesoterodine fumarate,
   (ii) 20-75% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof"
   (iii) 20-60% controlled release polymers, selected from a group consisting of hydroxypropyl methylcellulose, methylcellulose and mixture thereof,
   (iv) 2-5% glidants, preferably talc,
   (v) 2-5% lubricants, preferably glyceryl behenate.
17. The dry formulation according to any of preceding items further comprising
   (i) 2-3% fesoterodine fumarate,
   (ii) 40-55% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof,
   (iii) 40-50% controlled release polymers, selected from a group consisting of hydroxypropyl methylcellulose, methylcellulose and mixture thereof,
   (iv) 2-3% glidants, preferably talc,
   (v) 3-4% lubricants, preferably glyceryl behenate.
18. A pharmaceutical composition, obtained from a dry formulation of fesoterodine or a salt or a solvate thereof according to any one of the preceding items by any one of direct compression or dry granulation followed by compression of obtained dry granulate.
19. The pharmaceutical composition according to item 18, provided in the form of tablets or in the form of powder mixture or granules filled in capsules or in sachets.
20. The pharmaceutical composition according to item 18, wherein said direct compression or dry granulation forms tablet cores, which are respectively coated by a coating.
21. The pharmaceutical composition according to item 20, wherein a coating is provided and comprises an excipient selected from the group consisting of polyvinyl alcohol, hydroxypropyl methylcellulose and hydroxypropyl cellulose.
22. A process for preparing a dry formulation according to any one of items 1 to 17, comprising mixing fesoterodine or a salt or a solvate thereof with at least one of said pharmaceutically acceptable excipient under a condition of absence of any liquid or in the presence of maximally 10% liquid.
23. The process according to item 22, comprising:
   (i) mixing fesoterodine or a salt or a solvate thereof with filler selected selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof,
      glidant, preferably talc,
      lubricants, preferably glyceryl behenate; and
      optionally controlled release polymers selected from a group consisting of hydroxypropyl methylcellulose, methylcellulose and mixture thereof,
   (ii) optionally sieving the mixture.
24. The process according to item 22 or 23 further comprising direct compression of dry formulation into tablet cores.
25. The process according to item 22 or 23 further subjecting the dry formulation to dry granulation or moisture-activated dry granulation and compressing the obtained dry granulate into tablet cores.
26. The process according to item 25, wherein the mixture is subjected to a granulation step comprising
   (i) compacting the dry formulation into compacts with roller compactor or tabletting machine by using slugging or tableting tooling,
   (ii) milling the obtained compacts into dry granulate.
27. A process for preparing a pharmaceutical composition comprising preparing a dry formulation according to any of the items 22 to 26, wherein the process uses direct compression or dry granulation.
28. The process according to item 27, wherein the mixture is compressed or granulated and tableted into tablet cores, and the cores are coated with coating containing polymer selected from the group consisting of polyvinyl alcohol, hydroxypropyl methylcellulose, hydroxypropyl cellulose.
29. The dry formulation according to any one of items 1 to 17, or the pharmaceutical composition according to any of the items 18 to 21, for use in the method of treatment of urinary incontinence.
30. The dry formulation according to any one of items 1 to 17, or the pharmaceutical composition according to any of the items 18 to 21, for use in the preparation of a medicament for the treatment of urinary incontinence.
31. Any one of the preceding items, wherein the fesoterodine salt is fesoterodine fumarate.

### Description of further advantages and preferred embodiments of the invention

The present invention is now described in more detail by preferred embodiments and examples, which are however presented for illustrative purpose only.

The object of the present invention was to provide an improved system with a chemically stable fesoterodine or a salt or a solvate thereof as well as a more robust, economical and acceptable production process thereof.

In one aspect the present invention provides a dry formulation of fesoterodine or a salt or a solvate thereof, comprising fesoterodine or a salt or a solvate thereof homogeneously admixed with at least one excipient in said dry formulation, and wherein said dry formulation is free of a stabilizer selected from xylitol, sorbitol, polydextrose, isomalt and dextrose.

It was surprisingly found that fesoterodine or a salt or solvate thereof showed an improved stability of fesoterodine, when its dry formulation was prepared in homogeneous admixture with at least one excipient.

Further surprisingly, the dry formulation according to the present invention disclosed herein shows even improved stability of fesoterodine compared to a comparison product containing stabilizers which were found best in WO 2007/141298. Therefore, when desired that the use of such stabilizers selected from the group consisting of xylitol, sorbitol, polydextrose, isomalt and dextrose can be dispensed with, which in turn means that at least the dry formulation itself and hence tablet cores or granules obtained therefrom can be made free of said specified stabilizers. It is believed that the presence of a homogeneous admixture of pharmaceutically suitable excipient, combined with the proviso that a dry formulation in the essential or total absence of liquid is provided, establishes a protective matrix for the labile active compound. Moreover, since the dry formulation provided by the present invention allows to avoid a drying step or a step including heating or a step creating moisture conditions, further potential negative impacts affecting stability of fesoterodine, its salt or solvate can be reduced by the present invention.

The term "dry formulation" used herein means a preparation wherein the active principle fesoterodine or a salt or a solvate thereof and further ingredients for a pharmaceutical dosage form, especially tablets, have been processed while avoiding typical wet conditions, but using essentially dry or completely dry conditions. This is typically made by using limited amounts of liquids during processing of the active principle, for example to a range up to maximally 10 wt.-%, preferably clearly below 10 wt.-%, more preferably lower than 5 wt.-% and particularly lower than 2 wt.% of the whole formulation mass, or preferably totally avoiding liquids otherwise normally used for agglomeration in wet granulation, such as for example water, ethanol or their combination. Accordingly, it is preferred that residual amount of water/moisture in the dry formulation of the present invention is 4.0 wt.-% or below, preferably 2 wt.-% or below, respectively measured by loss on drying for 15 minutes at 80°C. Stabilization of fesoterodine, its salt or solvate within the dry formulation is particularly efficient when the content of inactive excipients constitutes the major amount, i.e. more than 50%, of the homogeneous admixture in the total dry formulation, preferably at least 75%, particularly at least 90% the total dry formulation. It is believed that addition of such substantial amount of excipients into a common dry granulate to thereby provide a homogeneous admixture significantly contributes to stabilization of the fesoterodine compound even in the absence of a special stabilizer, i.e. distinct from a situation where only a special stabilizer substance (as described in WO2007/141298) is added into a granulation of fesoterodine or its salt or solvate.

In a preferred embodiment the active ingredient of fesoterodine is the salt fesoterodine fumarate.

In a further aspect the present invention provides a dry formulation comprising fesoterodine or a salt or a solvate thereof, wherein the dry formulation is obtainable by a process carried out substantially or even entirely without the presence of liquid.

The term "liquid" used herein refers to a material in a liquid state. Typically, the liquid meant is water or lower alcohol such as methanol or ethanol or aqueous mixtures, especially water.

In another aspect, the present invention further provides a pharmaceutical composition that is obtainable from the afore-defined dry formulation by direct compression or dry granulation followed by compression of dry granulate. In this aspect, the applied technique of direct compression or dry granulation followed by compression of dry granulate may form tablet cores. Dry formulation or dry granulate can be filled in capsules or in sachets. Tablet cores can then optionally be respectively coated by a coating.

The term "direct compression" used herein means a preparation wherein dry formulation containing the active principle fesoterodine or a salt or a solvate thereof and further ingredients for a pharmaceutical dosage form have been processed by applying of a sufficient force by the punches of a tablet press on a powder to compact it into a tablet.

The term "dry granulation" used herein means a preparation wherein the dry formulation containing the active principle fesoterodine or a salt or a solvate thereof and further ingredients for a pharmaceutical dosage form have been processed by compacting into compacts with roller compactor or tabletting machine by using slugging tooling or regular tableting tooling and subsequently milling - crushing and sizing these compacts into dry granulate.

The present invention further provides a set of samples of the dry formulation, or of the pharmaceutical composition according to the previous aspects, respectively further comprising pharmaceutically acceptable excipients, such as fillers, glidants and lubricants.

The dry formulation or the pharmaceutical composition described herein can further contain fillers and/or binders such as lactose monohydrate, microcrystalline cellulose, powdered cellulose, compressible sugar, starch (e.g., corn starch or potato starch), pregelatinized starch, fructose, sucrose, dextrose, dextranes, other sugars such as sucrose, mannitol, siliconized microcrystalline cellulose, calcium hydrogen phosphate, calcium hydrogen phosphate dihydrate, dicalciumphosphate dihydrate, tricalciumphosphate, calcium lactate and composed fillers of microcrystalline cellulose and lactose monohydrate, composed fillers of powdered cellulose and lactose monohydrate, composed fillers of corn starch and lactose monohydrate, and combinations thereof. The fillers may be present in the form of a single compound or in the form of a mixture of compounds.

Preferably, further excipients include at least one filler selected a group of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate. Preferably, a mixture of lactose and microcrystalline cellulose in a ratio of about 1:1 to about 4:1 [w/w] is used as the filler. A particularly preferred further excipient is MICROCELAC® 100, which is a co-processed mixture of lactose monohydrate and microcrystalline cellulose in a ratio of 3:1. Both the filling properties of lactose and the binding capacity of microcrystalline cellulose are synergistically co-processed to one excipient providing improved flow properties and better tabletting performance to the composition.

The dry formulation or the pharmaceutical composition described herein may also comprise binders, such as cellulose derivatives (e.g., methylcellulose and sodium carboxymethylcellulose), gelatin, glucose, lactose, sucrose, polyethylene glycol, polymethacrylates, hydroxypropylcellulose, sugar alcohols, pregelatinized starch and sodium alginate. The binder may be present in the form of a single compound or in the form of a mixture of compounds.

The dry formulation or the pharmaceutical composition described herein may also comprise glidants, such as starches, colloidal silicon dioxide and talc. The term "glidants" as used herein is defined as an agent improving the fluidity of the powder and thus the filling of the compression chamber of the tablet press. The gliding agent may be present in the pharmaceutical composition in the form of a single compound or in the form of a mixture of compounds. In a preferred embodiment glidant is talc.

The dry formulation or the pharmaceutical composition described herein may also comprise lubricants. The term "lubricant" as used herein is defined as an agent able to decrease adhesion of a powder to punches and friction between particles. The lubricant may be present in the pharmaceutical composition in the form of a single compound or in the form of a mixture of compounds. Various suitable lubricants include but are not limited to stearic acid, talc, hydrogenated vegetable oil (*e.g.* hydrogenated castor oil), sodium lauryl sulphate, glyceryl behenate, polyethylene glycol, magnesium stearate and sodium stearyl fumarate. In a preferred embodiment lubricant is selected from the group consisting of hydrogenated castor oil, polyethylene glycol, glyceryl behenate, magnesium stearate and sodium stearyl fumarate, more preferably lubricant glyceryl behenate, sodium stearyl fumarate, magnesium stearate, calcium stearate or stearic acid. Most preferably the lubricant is glyceryl behenate.

The present invention further provides a set of samples of dry formulation or of pharmaceutical composition according to the previous aspects, respectively comprising: about 0.5-15% fesoterodine or a salt or a solvate thereof,
about 5-99% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
about 0.5-5% glidants and
about 0.5-5% lubricants.

The term "about" generally means within 10%, preferably 5% and more preferably within 1% of a given value or range. Alternatively, the term "about" means within an acceptable standard error of the mean, when considered by one of the ordinary skill in the art.

In one embodiment the dry formulation or the pharmaceutical composition comprises
about 2-5% fesoterodine or a salt or a solvate thereof,
about 20-95% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
about 2-5% glidants and
about 2-5% lubricants.

In a preferred embodiment the dry formulation or the pharmaceutical composition comprises about 2-3% fesoterodine or a salt or a solvate thereof,
about 40-95% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
about 2-3% glidants and
about 3-4% lubricants.

In another aspect, the present invention provides a the dry formulation, or the pharmaceutical composition further comprise a polymer as an excipient homogeneously admixed with the fesoterodine or a salt or a solvate thereof in said dry formulation.

According to one embodiment, the polymer may be a fast dissolving polymer. Suitable fast dissolving polymers include, without being limited thereto, polyethylene glycol 2000 - 6000, polyvinylpyrrolidone, polyvinyl alcohol-polyethylene glycol graft copolymer, polyoxyethylene copolymers, polyoxypropylene copolymers, polyethyleneoxide, hydroxypropyl cellulose, hydroxyethyl cellulose, hydroxyalkyl alkali metal carboxyalkylcellulose derivatives, hydroxyethyl carboxymethyl cellulose, hydroxymethyl carboxyethyl cellulose, hydroxymethyl carboxymethyl cellulose, hydroxypropyl carboxymethyl cellulose, hydroxypropyl carboxyethyl cellulose, hydroxypropyl carboxyethyl cellulose, hydroxypropyl carboxypropyl cellulose, hydroxybutyl carboxymethyl cellulose; or a mixture of any of these compounds.

According to another, preferred embodiment, the polymer is a controlled release polymer. Suitable controlled release polymers include, without being limited thereto, polyvinylpyrrolidone, polymethacrylate, polyvinylacetate, dextranes, starch, cellulose ethers and esters like methylcellulose, methylethylcellulose, hydroxyethylcellulose, hydroxypropylmethylcellulose, hydroxypropylcellulose, or carboxymethylcellulose, or mixtures thereof. According to a further preferred embodiment, the controlled release polymer hydroxypropyl methylcellulose.

In a further preferred embodiment the pharmaceutical composition comprises two different types of controlled release polymers, respectively selected from the group consisting of at least two viscosity grades of hydroxypropyl methylcellulose.

For example, the particularly preferred controlled release polymers are selected from a group of hydroxypropyl methylcellulose (HPMC), methylcellulose and mixture thereof. HPMC is preferably present in an amount that allows for the formation of a gel matrix from which the active ingredient is gradually released. Particularly preferred brands are METHOCEL® K100M having a nominal viscosity of about 100,000 mPas and METHOCEL® K4M having a nominal viscosity of about 4,000 mPas. The weight ratios of METHOCEL® K100M and K4M used in the compositions described herein can be in the range of about 20:1 to about 1:20, and are preferably in the range of about 10:1, and are even more preferably in the range of about 5:1.

Preferably, the above defined samples of dry formulation or of pharmaceutical composition according to anyone of the preceding embodiments comprise 15-65% of the polymer excipient, preferably of the controlled release polymer, relative to the total amount of the dry formulation. In the most preferred embodiment the pharmaceutical composition comprises about 40-50% polymer.

In another embodiment, the dry formulation or the pharmaceutical composition comprises about 0.5-10% fesoterodine fumarate,
about 5-85% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
about 15-65% controlled release polymers, selected from a group consisting of hydroxypropyl methylcellulose, methylcellulose and mixture thereof,
about 0.5-5% glidants, preferably talc,
about 0.5-5% lubricants, preferably glyceryl behenate.

In a preferred embodiment, the dry formulation or the pharmaceutical composition comprises about 2-5% fesoterodine fumarate,
about 20-75% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
about 20-60% controlled release polymers, selected from a group consisting of hydroxypropyl methylcellulose, methylcellulose and mixture thereof,
about 2-5% glidants, preferably talc,
about 2-5% lubricants, preferably glyceryl behenate.

In a particularly preferred embodiment, the dry formulation or the pharmaceutical composition comprises
about 2-3% fesoterodine fumarate,
about 40-55% filler, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
about 40-50% controlled release polymers, selected from a group consisting of hydroxypropyl methylcellulose, methylcellulose and mixture thereof,
about 2-3% glidants, preferably talc,
about 3-4% lubricants, preferably glyceryl behenate.

In a further aspect, the pharmaceutical composition further comprises a coating using a polymer selected from the group consisting of polyvinyl alcohol, hydroxypropyl methylcellulose, hydroxypropyl cellulose, hydroxyethylcellulose and polymethacrylates.

The term "coating" as used herein refers to a layer which completely covers an object and is applied by film coating. The coating can be selected from the group of ready to form dispersion such as OPADRY. The coating dispersion may comprise hydrophilic film forming polymer (such as for example low viscosity HPMC, HPC, PVA (polyvinylalcohol) and the like), plastificators (*e.g.* PEG), colorants and may optionally include other excipients such as antitacking agents.

Any method for film coating, known in the field of the pharmaceutical technology, may be used.

A pharmaceutical composition according to the present invention is preferably in solid form, including tablets, capsules, caplets, lozenges and sachets. Tablets may be suitably coated (film coated tablets, pills). Capsule formulations may cover both soft and hard capsules. A pharmaceutical compositions according to the present invention is preferably in the form of tablet, more preferably coated tablet with appropriate film coating material.

In another aspect, the present invention further provides a process for preparing the pharmaceutical composition comprising fesoterodine or a salt or a solvate thereof.

In a preferred embodiment the process is carried out substantially without the presence of liquid.

The prior art teaches wet granulation as the preferred process for preparation of pharmaceutical composition comprising fesoterodine, as higher degree of degradation of fesoterodine was found by using dry granulation process. Surprisingly, we have observed that using the direct compression or dry granulation process followed by compression of the dry granulate with appropriate excipient gives satisfactory chemical stability of fesoterodine or a salt or a solvate thereof in the resulting pharmaceutical composition, even without special stabilizer as disclosed in WO2007/141298, and at the same time dissolution profiles and variability of final product are acceptable. Moreover, performing energy consuming drying in the production of the tablets can be beneficially omitted in the course of simply using direct compression, or dry granulation followed by compression of the dry granulate. When using dry granulation followed by compression of the dry granulate, the process of the present invention may include only a low number of steps such as, e.g blending, optionally sieving, tableting or briquetting or slugging, and tabletting. Especially when using direct compression, the process of the present invention may include even lower number of steps such as e.g blending, optionally sieving, tabletting, which is in favor of process economy and costs. This together with selecting appropriate excipients and additives as described above makes the whole process of the invention more robust, economical and acceptable.

In one aspect the process according to the present invention comprises mixing fesoterodine or a salt or a solvate thereof with at least one pharmaceutically acceptable excipient, and compressing the mixture or subjecting the mixture to dry granulation or moisture-activated dry granulation, followed by compression of dry granulate wherein the mixture can be devoid of any substance selected from xylitol, sorbitol, polydextrose, isomalt and dextrose. The term "moisture-activated dry granulation" (MADG), as described for example by Christensen et al. in "Drug Development and Industrial Pharmacy" 20(14), pp. 2195-2213 (1994) is based on the addition of limited amount of water to a dry blend of the active compound, binder and filler without the need of a drying step being involved. The use of non-aqueous liquid or aqueous mixtures thereof instead of limited water in an otherwise dry mixing system ("non-aqueous moisture activated dry granulation"; NAMADG) is also possible. Non-aqueous liquids may include alcohols such as ethanol, higher alcohols, oils, liquid glycols such as liquid polyethylene glycols, etc.

In a preferred embodiment the process comprises
mixing fesoterodine or a salt or a solvate thereof with fillers, selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
   glidants, preferably talc,
   lubricants, preferably glyceryl behenate and
   optionally polymers, specifically controlled release polymers selected from a group
consisting of hydroxypropyl methylcellulose, methylcellulose and mixture thereof, optionally sieving the mixture.

In another aspect, the present invention further provides a process for preparing the pharmaceutical composition from dry formulation further comprising at least one dry granulation step, optionally followed by tabletting, wherein the dry formulation can be devoid of any substance selected from xylitol, sorbitol, polydextrose, isomalt and dextrose.

In yet another aspect, the present invention further provides a process wherein the dry formulation is subjected to a dry granulation step comprising:
compacting the dry formulation into compacts with roller compactor or tabletting machine by using slugging or tableting tooling,
milling the obtained compacts into dry granulate.

The advantages of direct compression and dry granulation over wet granulation include, less manufacturing steps involved, elimination of heat and moisture. The overall process of direct compression may involve only weighing of the powders, optionally sieving, blending, and finally compression, hence less cost than wet granulation. The overall process of dry granulation followed by the compression of dry granulate may involve only weighing of the powders, briquetting or slugging, optionally sieving, milling and blending, and finally compression, hence also less cost than wet granulation.

In most preferred embodiment the granulation step comprises milling of the compacts obtained by compressing the dry formulation, and compressing the obtained dry granulate.

In another aspect, the present invention further provides a process for preparing the pharmaceutical composition further comprising coating of the compressed mixture with a coating mixture containing polymer selected from the group consisting of polyvinyl alcohol, hydroxypropyl methylcellulose, hydroxypropyl cellulose.

In another aspect, the present invention further provides the dry formulation or the pharmaceutical composition according to the present invention for use in a method of treatment of urinary incontinence.

In the preferred embodiment the pharmaceutical formulation is used in the preparation of a medicament for the treatment of urinary incontinence.

### Examples

| Comparative example- prepared according to WO2007/141298 (Example G) | | | |
|---|---|---|---|
| Component | Amount per tablet (mg) | Portion | Amount per lab. batch (g) |
| GRANULATE | | | |
| Fesoterodine fumarate | 8.000 | 2.5% | 8.000 |
| Xylitol | 72.000 | 22.5% | 72.000 |
| Total | 80.000 | 25.0% | 80.000 |
| TABLETS | | | |
| Granulate | 80.000 | 25.0% | 40.000 |
| Lactose monohydrate | 58.125 | 18.2% | 29.063 |
| Microcrystalline cellulose | 19.375 | 6.1% | 9.688 |
| Hypromellose Methocel K100M | 120.000 | 37.5% | 60.000 |
| Hypromellose Methocel K4M | 24.000 | 7.5% | 12.000 |
| Gliceryl Behenate | 10.000 | 3.1% | 5.000 |
| Talc | 8.500 | 2.7% | 4.250 |
| Total | 320.000 | 100.0% | 820.000 |

Fesoterodine fumarate and xylitol were granulated with demineralized water manually in mortar with pestle. Granulate was dried in vacuum chamber drier to LOD 0.5%. Dried granulate was pushed through 0.8 mm screen. Granulate was mixed with lactose and microcrystalline cellulose and passed through screen 0.8 mm. Then Methocel K4M and Methocel K100M were added to the screened mixture and blended in double-cone blender for 5 min. The mixture was then again passed through screen 0.8 mm and blended in double-cone blender for 10 min so that final tabletting blend was obtained. Final blend was compressed into oval biconvex tablets by rotary tabletting machine.

| Example 1 | | | |
|---|---|---|---|
| Component | Amount per tablet (mg) | | Amount per batch (g) |
| Fesoterodine fumarate | 8.000 | 2.5% | 1.600 |
| Microcrystalline cellulose | 50.000 | 15.6% | 10.000 |
| Lactose monohydrate | 100.000 | 31.3% | 20.000 |
| Hypromellose Methocel K100M | 120.000 | 37.5% | 24.000 |
| Hypromellose Methocel K4M | 24.000 | 7.5% | 4.800 |
| Gliceryl Behenate | 10.000 | 3.1% | 2.000 |
| Talc | 8.000 | 2.5% | 1.600 |
| Total | 320.000 | 100.0% | 64.000 |

Fesoterodine fumarate was manually mixed with microcrystalline cellulose and lactose in polyethylene bag for 1 min. Obtained blend was then passed through hand sieve 0.5 mm. Methocel K4M and Methocel K100M were added to screened blend and manually mixed in polyethylene bag for 3 min. Then talc was added to the blend in PE bag, and mixed for 1 min. Then glyceryl behenate was added to the blend in PE bag and mixed for 1 min, so that final tabletting blend was obtained. Final blend was compressed into oval biconvex tablets by eccentric tabletting machine.

| Example 2 | | | |
|---|---|---|---|
| Component | Amount per tablet (mg) | | Amount per lab. batch (g) |
| Fesoterodine fumarate | 8.000 | 2.5% | 60.000 |
| Lactose monohydrate | 100.000 | 31.3% | 750.000 |
| Microcrystalline cellulose | 50.000 | 15.6% | 375.000 |
| Hypromellose Methocel K100M | 120.000 | 37.5% | 900.000 |
| Hypromellose Methocel K4M | 24.000 | 7.5% | 180.000 |
| Gliceryl Behenate | 10.000 | 3.1% | 75.000 |
| Talc | 8.000 | 2.5% | 60.000 |
| Total | 320.000 | 100.0% | 2400.000 |

Fesoterodine fumarate was manually mixed with lactose in polyethylene bag for 1 min to obtain Blend 1. Blend 1 was then passed through 0.5 mm screen on oscillating bar mill. Screened Blend 1 was then transferred to bin blender (double-cone blender) and blended for 2 min. Microcrystalline cellulose, Methocel K4M and Methocel K100M were then added to Blend 1 in bin blender and blended for additional 2 min to obtain Blend 2. Blend 2 was then passed through 0.5 mm screen on oscillating bar mill and transferred back to double-cone blender and blended for additional 2 min. Then talc was added to the blend in double-cone blender.and mixed for 2 min. Then glyceryl behenate was added to the blend in double-cone g and mixed for 1 min so that final tabletting blend was obtained. Final blend was compressed into oval biconvex tablets by rotary tabletting machine.

| Example 3 | | | |
|---|---|---|---|
| Component | Amount per tablet (mg) | | Amount per lab. batch (g) |
| Fesoterodine fumarate | 8.000 | 2.5% | 60.000 |
| Lactose monohydrate | 100.000 | 31.3% | 750.000 |
| Microcrystalline cellulose | 50.000 | 15.6% | 375.000 |
| Hypromellose Methocel K100M | 120.000 | 37.5% | 900.000 |
| Hypromellose Methocel K4M | 24.000 | 7.5% | 180.000 |
| Gliceryl Behenate | 10.000 | 3.1% | 75.000 |
| Talc | 8.000 | 2.5% | 60.000 |
| Total | 320.000 | 100.0% | 2400.000 |

Fesoterodine fumarate was manually mixed with lactose in polyethylene bag for 1 min to obtain Blend 1. Blend 1 was then passed through 0.5 mm screen on oscillating bar mill. Screened Blend 1 was then transferred to bin blender (double-cone blender) and blended for 2 min. Microcrystalline cellulose, Methocel K4M and Methocel K100M were then added to Blend 1 in bin blender and blended for additional 2 min to obtain Blend 2. Blend 2 was then passed through 0.5 mm screen on oscillating bar mill and transferred back to double-cone blender and blended for additional 2 min. Then talc was added to the blend in double-cone blender, and mixed for 2 min. Then glyceryl behenate was added to the blend in double-cone g and mixed for 1 min, so that final tabletting blend was obtained. Final blend was compressed into oval biconvex tablets by rotary tabletting machine.

Obtained tablets were milled by impact hammer mill with blades and screen 1.0 mm. Obtained dry granulate was compressed into oval biconvex tablets by rotary tabletting machine.

| Example 4 | | | |
|---|---|---|---|
| Component | Amount per tablet (mg) | | Amount per lab. batch (g) |
| Tablet cores Example 3 | 320.000 | 95.5% | 800.000 |
| Opadry II | 15.000 | 4.5% | *75.000 |
| Demineralized water | 60.000 | | *300.000 |
| Total | 335.000 | 100.0% | 837.500 |

| | | | |
|---|---|---|---|
| *The coating dispersion was prepared in double amount, due to possible losses and the coating end was determined by weighing of coated tablets. | | | |

Tablet cores prepared as disclosed in Example 3 were film coated in perforated pan coater by spraying the coating dispersion onto tablet cores. The coating dispersion was prepared by dispersing of Opadry II in demineralized water.

| Example 5 | | | |
|---|---|---|---|
| Component | Amount per tablet (mg) | | Amount per lab. batch (g) |
| Tablet cores Example 3 | 320.000 | 95.5% | 800.000 |
| Hypromellose Pharmacoat 606 | 8.775 | 2.6% | *43.875 |
| Hydroxypropyl cellulose | 0.975 | 0.3% | *4.875 |
| Polyethylene glycol | 1.500 | 0.4% | *7.500 |
| Titanium dioxide | 2.400 | 0.7% | *12.000 |
| Blue Pigment Indigotin | 0.600 | 0.2% | *3.000 |
| Talc | 0.750 | 0.2% | *3.750 |
| Demineralized water | 135.000 | | *675.000 |
| Total | 335.000 | 100.0% | 837.500 |

| | | | |
|---|---|---|---|
| * The coating dispersion was prepared in double amount, due to possible losses and the coating end was determined by weighing of coated tablets. | | | |

Tablet cores prepared as disclosed in Example 3 were film coated in perforated pan coater by spraying the coating dispersion onto tablet cores. The coating dispersion was prepared by dispersing of Hypromellose, Hydroxypropyl cellulose, Polyethylene glycol, titanium dioxide, pigment and talc in demineralized water.

### Stress stability tests:

A stress stability test was designed to show intrinsic stability of test formulations in comparison to a reference formulation, known from prior art. The test samples (tablets) were exposed to elevated temperature (60°C) and a controlled relative humidity (21%). The appropriate relative humidity was achieved by means of a saturated solution of potassium fluoride, stored in an impermeable cabined inside a thermostatically controlled chamber, set at 60°C. Tablets were exposed to this stress condition for two weeks with no primary packaging (open dish). After two weeks, samples were analyzed for content of 5-HMT by means of high performance liquid chromatograph (HPLC).

HPLC method was run on an Alliance HPLC instrument (Waters) using BEH Shield RP18 column (50 x 2.1 mm 1.7 um particles). The mobile phase consisted of 0.05% phosphoric acid (A) and acetonitrile (B). The mobile phase was pumped at 0.5 ml/min with a gradient, beginning at 10% mobile phase B, increasing to 90% mobile phase B in 4 minutes, followed by column reequilibration for two minutes. Chromatograms were followed at 220 nm and typical retention times of fesoterodine fumarate and 5-HMT were 2.5 and 1.5 min, respectively. Evaluation is done by the Area% method

### Stability evaluation:

A formulation is preferred, if the amount of 5-HMT, formed after exposure to the indicated stress conditions, is below or equal to that of the comparative example.

| | % 5-HMT (hydrolysis product) | Total degradation products |
|---|---|---|
| Example 1 | 2.0 | 4.5 |
| Example 2 | 2.4 | 5.0 |
| Example 3 | 2.4 | 5.0 |
| Example 4 | 2.2 | 5.1 |
| Example 5 | 2.4 | 5.3 |
| Comparative example | 4.0 | 10.8 |

## Claims

1. A dry formulation of fesoterodine or a salt or a solvate thereof, comprising said fesoterodine or a salt or a solvate thereof homogeneously admixed with at least one excipient in said dry formulation, and wherein said dry formulation is free of a stabilizer selected from xylitol, sorbitol, polydextrose, isomalt and dextrose.

2. The dry formulation according to claim 1, wherein an optional liquid, when used, was limited during the dry formulation preparation process at a maximum of 10% liquid relative to the composition subjected to dry formulation.

3. The dry formulation according to anyone of the preceding claims, comprising pharmaceutically acceptable excipients selected from fillers, glidants and lubricants present in said dry formulation.

4. The dry formulation according to any of preceding claims, comprising relative to the total amount of the dry formulation:
(i) 0.5-15% fesoterodine or a salt or a solvate thereof,
(ii) 5-99% filler selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof,
(iii) 0.5-5 % glidant,
(iv) 0.5-5% lubricant.

5. The dry formulation according to any one of the preceding claims, wherein an excipient, homogeneously admixed with the fesoterodine or a salt or a solvate thereof in said dry formulation, is a polymer, preferably a controlled release polymer.

6. The dry formulation according to claim 5, wherein the controlled release polymer is selected from the group consisting of hydroxypropyl methylcellulose, methylcellulose hydroxyethylcellulose, hydroxypropylcellulose, carboxymethylcellulose, ethylcellulose, polyethyleneoxide, carrageenan, agar, alginic acid, pectin and a mixtures thereof, preferably hydroxypropyl methylcellulose.

7. The dry formulation according to claim 6, wherein the controlled release polymer is a combination of at least two different viscosity grades of hydroxypropyl methylcellulose.

8. The dry formulation according to any of claims 5 to 7, comprising relative to the total amount of the dry formulation:
(i) 0.5-10% fesoterodine fumarate,
(ii) 5-85% filler selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof
(iii) 15-65% controlled release polymers, selected from a group consisting of hydroxypropyl methylcellulose, methylcellulose and mixture thereof,
(iv) 0.5-5% % glidant, preferably talc,
(v) 0.5-5% % lubricant, preferably glyceryl behenate.

9. A pharmaceutical composition, obtained from a dry formulation of fesoterodine or a salt or a solvate thereof according to any one of the preceding claims by direct compression or dry granulation followed by compression of dry granulate.

10. The pharmaceutical composition according to claim 9, wherein said direct compression or dry granulation followed by compression of dry granulate forms tablet cores, which are respectively coated by a coating.

11. The pharmaceutical composition according to claim 10, wherein a coating comprises a polymer excipient selected from the group consisting of polyvinyl alcohol, hydroxypropyl methylcellulose and hydroxypropyl cellulose.

12. A process for preparing a dry formulation according to any one of claims 1 to 8, comprising mixing fesoterodine or a salt or a solvate thereof with at least one of said pharmaceutically acceptable excipient under a condition of absence of any liquid or in the presence of maximally 10% liquid.

13. The process according to claim 12, comprising:
(i) mixing fesoterodine or a salt or a solvate thereof with filler selected from a group consisting of microcrystalline cellulose, lactose monohydrate, corn starch, calcium phosphate, calcium hydrogenphosphate, sucrose, mannitol, composed fillers of microcrystalline cellulose and lactose monohydrate, composed fillers of powdered cellulose and lactose monohydrate, composed fillers of corn starch and lactose monohydrate, and combinations thereof,
lubricants, preferably glyceryl behenate; and
optionally controlled release polymers selected from a group consisting of hydroxypropyl methylcellulose, methylcellulose and mixture thereof, and
(ii) optionally sieving the mixture.

14. The process according to claim 12 or 13 further comprising direct compression of dry formulation into tablet cores.

15. The process according to claim 12 or 13 further subjecting the dry formulation to dry granulation or moisture-activated dry granulation and compressing the obtained dry granulate into tablet cores.

16. The process according to claim 15, wherein the dry formulation is subjected to a dry granulation step comprising:
(i) compacting the dry formulation into compacts with roller compactor or tabletting machine by using slugging or tableting tooling,
(ii) milling the obtained compacts into dry granulate.

17. The process according to any of claims 12 to 16, wherein the mixture is compressed or dry granulated and tabletted into tablet cores, and the cores are coated with coating, preferably the coating contains a polymer selected from the group consisting of polyvinyl alcohol, hydroxypropyl methylcellulose, hydroxypropyl cellulose.

18. The dry formulation according to any one of claims 1 to 8, or the pharmaceutical composition according to any of the claims 9 to 11 for use as a medicament, preferably for use in a treatment of urinary incontinence.
